# Europäisches Patentamt

## European Patent Office

## Office européen des brevets

(11) Veröffentlichungsnummer: **0 007 412**
**B1**

(12) # EUROPÄISCHE PATENTSCHRIFT

(45) Veröffentlichungstag der Patentschrift:
25.02.87

(51) Int. Cl.⁴: **A 61 K 31/415**

(21) Anmeldenummer: 79101874.0

(22) Anmeldetag: 11.06.79

(54) **Verwendung von 2-(N-(2,6-Dichlorphenyl)-N-allyl-amino)-imidazolin-(2) zur Herstellung von bradykarden Arzneimitteln.**

(30) Priorität: 15.07.78 DE 2831190

(43) Veröffentlichungstag der Anmeldung:
06.02.80 Patentblatt 80/3

(45) Bekanntmachung des Hinweises auf die Patenterteilung:
25.02.87 Patentblatt 87/9

(84) Benannte Vertragsstaaten:
AT BE CH DE FR GB IT LU NL SE

(56) Entgegenhaltungen:
BE-A-759 125
DE-A-2 404 754

Arzneimittelforschung, 25 (1975), 786-93
Arzneimittelforschung, 16 (1966), 1038-50
Arch. int. Pharmacodyn, 228 (1977), 237-50
Arch. int. Pharmacodyn, 228 (1977), 251-67
Hypertension and brain mechanisms. Progress in brain research. W. de Jong et al., Eds., Vol. 47, 391-96 (Elsevier/North-Holland Biomedical Press, Amsterdam, 1977)
J. Med. Chem., 23 (1980), 1217-22
Naunyn-Schmiedeberg's Archives of Pharmacology, 307, Suppl. 1979, p. R40, R41
Naunyn-Schmiedeberg's Archives of Pharmacology, 308, Suppl. 1979, p. R19
European Journal of Pharmacology, 58 (1979), 141-50
Naunyn-Schmiedeberg's Archives of

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim (DE)**
Erfinder: **Köppe, Herbert, Dr., Neuweg 72, D-6507 Ingelheim (DE)**
Erfinder: **Kummer, Werner, Dr., Georg- Scheuing-Strasse 15, D-6507 Ingelheim (DE)**
Erfinder: **Arndts, Dietrich, Dr., Mühlstrasse 7, D-6531 Appenheim (DE)**
Erfinder: **Kobinger, Walter, Prof. Dr., Belghofergasse 27, A-1120 Wien (AT)**
Erfinder: **Lillie, Christian, Dr., Hansi- Niese- Weg 12, A-1130 Wien (AT)**
Erfinder: **Pichler, Ludwig, Dr., Gusshausstrasse 24, A-1040 Wien (AT)**

(56) Entgegenhaltungen: (Fortsetzung)
**Pharmacology, 306 (1979), 255-62**

**Die Akte enthält technische Angaben, die nach dem Eingang der Anmeldung eingereicht wurden und die nicht in dieser Patentschrift enthalten sind.**

## Beschreibung

Die Erfindung betrifft die Verwendung eines Imidazolin-Derivates zur Herstellung eines Arzneimittels zur Reduzierung der Herzschlagfrequenz. Insbesondere betrifft sie die Verwendung von 2-[N-(2,6-Dichlorphenyl)-N-allyl-amino]-imidazo-lin-(2) und dessen Säureadditionssalze zur Herstellung eines Arzneimittels zur Prophylaxe und Therapie ischämischer Herzerkrankungen und von Sinustachykardien verschiedener Genesen.

Es ist bekannt, daß 2-[N-(2,6-Dichlorphenyl)-N-allyl-amino]-imidazolin-(2) und dessen Säureadditionssalze wertvolle pharmakodynamische Eigenschaften besitzen. Die Substanz, ihre Herstellung und Verarbeitung zu pharmazeutischen Anwendungsformen ist zum Beispiel in der belgischen Patentschrift Nr. 759 125 beschrieben, diese Patentschrift offenbart auch die analgetische und blutdrucksenkende Eigenschaft dieser Verbindung.

Es wurde nun gefunden, daß 2-[N-(2,6-Dichlorphenyl)-N-allyl-amino]-imidazolin-(2) und dessen Säureadditionssälze eine Senkung der Herzfrequenz bewirken. Dieser Befund wurde sowohl durch pharmakologische als auch durch klinische Studien sichergestellt.

Die Herzfrequenz wurde nach intravenöser Gabe an narkotisierten Tieren gesenkt: Ratte ab 0,5 mg/kg, Katze ab 0,3 mg/kg, Hund 2,5 mg/kg. Die Verbindung senkte die Herzfrequenz auch am wachen Tier: Am Hund ist die Substanzwirkung (2,5 mg/kg intravenös) abhängig von der Ausgangsfrequenz; geringer Substanzeffekt an vagotonen Normaltieren, stärkere Effekte nach experimenteller Frequenzerhöhung nach Atropin-, Hydralazinvorbehandlung. An der wachen Ratte trat eine Frequenzsenkung ab 5 mg/kg p.o. ein.

Die meisten der anderen cardiovaskulären Parameter waren im Verhältnis zur bradykarden Wirkung nur mäßig verändert. Die Substanz war nach Ausschaltung des ZNS voll bradycard wirksam. An der Spinalratte bewirken 2,5 mg/kg i.v. eine Senkung der Herzfrequenz um 150 Schläge/Minute. Dies spricht für einen direkten Angriffspunkt am Herzen, was durch die Abnahme der Frequenz am isolierten, spontan schlagenden Meerschweinvorhof bestätigt wurde (EC30 = 2,9 µ g/ml). Am elektrisch stimulierten Vorhofspräparat trat eine negativ inotrope (EC30 = 155 µg/ml) und antiarrhymische Wirkung (Folgefrequenztest, EC50 = 100 µ g/ml) erst in 53 bzw. 34-facher Konzentration auf. Als Hinweis auf schwache α-Adrenozeptor-stimulierende Wirkung kann Blutdrucksteigerung an der Spinalratte betrachtet werden. Die bradycarde Wirkung von 2-[N-(2,6-Dichlorphenyl)-N-allyl-amino]-imidazolin-(2) beruht nicht auf Erregung von cholinergen Rezeptoren (keine Abschwächung des Effektes von der Substanz durch Atropin am Meerschweinchenvorhof). Die Substanz besitzt keine β-Adrenozeptorenblockierende Wirkung.

An der narkotisierten Katze wurde das Tripelprodukt aus systolischem Blutdruck x Herzfrequenz x linsventrikulärer Auswurfzeit deutlich und langdauernd gesenkt. Am Myocardischämietest (narkotisierte Katze, EKG-Veränderungen bei kurzzeitiger Coronarocclusion) wurden die Ischämiezeichen (Anhebung von ST und T) durch 2,5 und 5 mg/kg i.v. der Substanz deutlich und langdauernd ( > 1 Stunde) vermindert. Beide Befunde sind Hinweise auf eine substanzbedingte Verminderung des myocardialen Sauerstoffverbrauches.

Beobachtungen am wachen Hund zeigen bei 5 mg/kg i.v. keinerlei zentrale Nebenwirkungen.

Die vorliegenden Ergebnisse grenzen die Verbindung deutlich von anderen bradycard wirksamen Substanzen und Substanzgruppen, wie Clonidin, Antiarrhythmika, Calciumantagonisten vom Verapamiltyp, cholinerge Substanzen, β-Adrenorezeptorenblocker, ab Aufgrund der spezifischen Frequenzsenkung (Herzentlastung) und der experimentellen Befunde, die auf eine Verminderung des myocardialen Sauerstoffbedarfes hinweisen (Tripelprodukt, Myocardischämietest), empfiehlt sich die Anwendung der Verbindung bei chronischer Coronarinsuffizienz.

An einer der klinischen Untersuchungen nahmen ein sportlich trainierter und für untrainierte, gesunde freiwillige männliche Personen teil.

Die Wirkungen der Verbindung wurden aufgrund des folgenden Versuchsplanes ermittelt:

Vor und zwei Stunden nach der Gabe von 40 mg 2-[N-(2,6-Di-chlorphenyl)-N-allyl-amino]-imidazolin-(2) in Form von Gelatine-Steckkapseln mit je 20 mg Wirkstoff wurde mit den Versuchspersonen in nüchternem Zustand ein Belastungsversuch durchgeführt. Im Ruhezustand wurde das EKG aufgenommen und der Blutdruck gemessen. Dann wurde unter Belastung mit Hilfe eines Fahrradergometers wiederum das EKG registriert und der Blutdruck gemessen, wobei die Belastung bei 50 Watt begann und nach jeweils 3 Minuten um 25 Watt gesteigert wurde bis zu einer Endleistung von 150 Watt. Drei Minuten nach der Belastung wurden wiederum im Ruhezustand das EKG registriert und der Blutdruck gemessen. Die Herzfrequenz wurde über die EKG-Auswertung ermittelt. In den Ruhe-EKG's wurden Extremitäten und Brustwandableitungen, unter Belastung nur die Brustwandableitungen, registriert.

Die Herzfrequenz verminderte sich nach Einnahme des Wirkstoffs bei den fünf untrainierten Probanden in Ruhe um durchschnittlich 12,6 Schläge/min., unter der 50 - 150 Watt-Belastung im Mittel um 15,0 - 18,2 Schläge je Minute und nach 3 Minuten Erholung um durchschnittlich 14,4 Schläge je Minute gegenüber dem Vorwert. Die maximale Einzelabnahme betrug dabei 28 Schläge pro Minute. Bei einem der fünf Probanden trat in Ruhe keine Verminderung auf. Bei dem trainierten Probanden zeigten sich keine eindeutigen Verminderungen der Herzfrequenz nach Medikation gegenüber dem Vorwert. Es wurde lediglich eine maximale Senkung der Herzfrequenz um 5 Schläge pro Minute bei 100 Watt-Belastung festgestellt.

Bei zwei der untrainierten Probanden traten unter der Wirkung der untersuchten Verbindung im Stromkurvenverlauf der EKG's Veränderungen auf:

1. Bei einem Proband fielen vor Medikation im Ruhe-EKG T-Abflachungen linkspräkordial auf, die sich unter der Belastung wieder aufrichten und nach Medikation nicht mehr zu sehen waren.

2. Bei dem anderen Probanden waren vor der Substanzgabe eine und nach Einnahme des Präparates drei Extrasystolen je Minute nachweisbar.

Im EKG des trainierten Probanden fielen keine substanzbedingten Veränderungen auf.

Der systolische Blutdruck lag nach Substanzgabe bei den fünf untrainierten Probanden in Ruhe im durchschnitt 9 mm Hg, unter der 50 - 150 Watt-Belastung im Mittel um 8 - 15 mm Hg und nach der Erholung um durchschnittlich 11 mm Hg unter dem Vorwert. Die maximale Einzelabnahme betrug dabei 25 mm Hg. Bei zwei der fünf Probanden waren unter den höheren Belastungsstufen keine Abnahmen gegenüber dem Vorwert zu erkennen. Bei dem trainierten Probanden nahm der systolische Blutdruck nach der Medikation in Ruhe und nach der Erholung um je 20 mm Hg und unter den verschiedenen Belastungsstufen um 10 - 30 mm Hg gegenüber dem Vorwert ab.

Der diastolische Blutdruck fiel nach Einnahme der Verbindung lediglich unter der 50 Watt-Belastung bei den fünf untrainierten Probanden um 5 - 10 mm Hg und bei dem trainierten Probanden um 10 mmHg dem Vor ert ab. In Ruhe, unter den übrigen Belastungsstufen und nach der Erholung zeigten sich bei allen sechs Probanden keine eindeutigen substanzbedingten Veränderungen.

Unter der Medikation verspürten vier der fünf untrainierten Probanden eine leichte Müdigkeit. Einem Probanden fiel die Ergometrie-Belastung nach Einnahme des Präparates leichter, einem schwerer als vor Medikation. Der trainierte Proband gab unter der Einwirkung der Verbindung eine leichte Sedation, leichte Mundtrockenheit und eine größere Anstrengung bei Belastung als vor Medikation zu Protokoll.

In einer weiteren klinischen Prüfung wurde an 2 Probanden zu geeigneten Zeitpunkten der Blutdruck gemessen (Riva-Rocci), das EKG registriert (in Ruhe Extremitäten- und Brustwandableitungen, unter Belastung nur Brustwandableitungen) und daraus die Herzfrequenz errechnet:

Nach Bestimmung der Ruhewerte im Liegen wurden die Probanden am Fahrradergometer steigender Belastung ausgesetzt. Es wurde mit 25 bzw. 50 Watt begonnen, und nach jeweils 3 Minuten die Belastung um 25 Watt erhöht, bis zu 125 bzw. 150 Watt. 3 Minuten nach Belastungsende wurden "Erholungswerte" gemessen. Am 1. Versuchstag morgens erfolgte vorerst ein Kontrollversuch. Danach nahmen die Probanden 80 mg Wirkstoff per os ein. 2 Stunden später wurde der Belastungsversuch wiederholt. 3 Tage später wurden morgens 40 mg Wirkstoff eingenommen, 2 Stunden später erfolgte ein Belastungsversuch.

Bei beiden Probanden wurde die Herzfrequenz durch 2-[N-(2,6-Dichlorphenyl)-N-allyl-amino]-imidazolin, unabhängig von der Dosis, sowohl in Ruhe als auch unter Belastung gesenkt. Bei einer Person waren auch die Erholungswerte nach Substanzgabe erniedrigt. Der systolische Blutdruck wurde bei der anderen Versuchsperson in Ruhe, unter Belastung und während der Erholungsphase durch die Substanz in beiden Dosen gesenkt, der diastolische Blutdruck zeigte keine wesentlichen Veränderungen. Bei einem Probanden sank nach Substanzgabe (beide Dosen) der systolische Blutdruck in Ruhe markant ab. Bei steigender Belastung stieg der systolische Blutdruck jedoch an und erreichte bei 100 Watt etwa die Werte des Kontrollversuchs.

Der Erholungswert war nach 80 mg deutlich niedriger als im Kontrollversuch. Der diastolische Blutdruck war in Ruhe gesenkt, unter Belastung verhielt er sich uneinheitlich. Im EKG wurden nach Substanzgabe außer einer Sinusbradycardie keine Veränderungen beobachtet.

Die Substanz wurde von beiden Probanden praktisch symptomlos vertragen.

Aufgrund der guten Verträglichkeit von 2-[N-(2,6-Dichlorphenyl)-N;alkyl-amino]-imidazolin-(2) ist die Verwendung dieser Substanz für die Herstellung eines Arzneimittels zur Prophylaxe und Therapie ischämischer Herzerkrankungen und bei Patienten mit Sinustachycardien verschiedener Genese geeignet.

Beispiele zur Herstellung von Tabletten, Ampullen und Tropfen sind in der belgischen Patentschrift Nr. 759 125 beschrieben. Bevorzugt sind Wirkstoffmengen von 5 bis 50 mg.

## Patentansprüche

1. Verwendung von-2-[N-(2,6-Dichlorphenyl)-N-allyl-amino]-imidazolin-(2) und dessen Säureadditionssalze zur Herstellung eines Arzneimittels zur Prophylaxe und Therapie von ischämischen Herzerkrankungen und von Sinustachycardien.

2. Verwendung von 2-[N-(2,6-Dichlorphenyl)-N-allyl-amino]-imidazolin-(2) und dessen Säureadditionssalze zur Herstellung eines Arzneimittels zur Reduzierung der Herzschlagfrequenz.

3. Arzneimittel nach Anspruch 1 und/oder 2, dadurch gekennzeichnet, daß es den Wirkstoff in einer Menge von 5 bis 50 mg je Dosiseinheit enthält.

## Claims

1. Use of 2-(N-(2,6-dichlorophenyl)-N-allyl-amino]-imidazoline-(2) and the acid addition salts thereof for preparing a pharmaceutical composition for the prophylaxis and therapy of ischaemic heart disease and sinus tachycardia.

2. Use of 2-[N-(2,6-dichlorophenyl)-N-allyl-amino]-imidazoline-(2) and the acid addition salts thereof for preparing a pharmaceutical composition for reducing the frequency of heart beats.

3. Pharmaceutical composition as claimed in claim 1 and/or 2, characterised in that it contains the active substance in an amount of from 5 to 50 mg per dosage unit.

## Revendicaitons

1. Utilisation de la 2-(N-(2,6-dichlorophényl)-N-allyl-amino)-imidazoline-(2) et de ses sels d'addition d'acides pour la préparation d'un médicament pour la prophylaxie et la thérapie des maladies cardiaques ischémiques et des tachycardies sinusales.

2. Utilisation de la 2-(N-(2,6-dichlorophényl)-N-allyl-amino]-imidazoline-(2) et de ses sels d'addition d'acides pour la préparation d'un médicament pour réduire la fréquence de battement du coeur.

3. Médicament selon la revendication 1 et/ou 2, caractérisé en ce qu'il contient la substance active en une quantité de 5 à 50 mg par dose unitaire.